## Europäisches Patentamt

## European Patent Office

## Office européen des brevets

(19)

(11) Veröffentlichungsnummer: **0 087 617**

**A1**

(12) # EUROPÄISCHE PATENTANMELDUNG

(21) Anmeldenummer: 83101151.5

(22) Anmeldetag: 07.02.83

(51) Int. Cl.³: **A 61 N 1/08**

(30) Priorität: 26.02.82 DE 3207050

(43) Veröffentlichungstag der Anmeldung:
07.09.83 Patentblatt 83/36

(84) Benannte Vertragsstaaten:
AT CH DE FR GB IT LI SE

(71) Anmelder: SIEMENS AKTIENGESELLSCHAFT
Berlin und München Wittelsbacherplatz 2
D-8000 München 2(DE)

(72) Erfinder: Kusserow, Bernd
Schallershofer Strasse 64
D-8520 Erlangen(DE)

(72) Erfinder: Strahwald, Franz
Pretzfelder Strasse 3
D-8553 Ebermannstadt(DE)

(72) Erfinder: Janz, Wolfgang
Am Vogelherd 128
D-8520 Erlangen(DE)

(54) **Medizinisches Gerät für die physikalische Therapie, insbesondere elektromedizinisches Reizstromgerät.**

(57) Bei bekannten Reizstromgeräten werden vom Therapeuten die Reizstromimpulsparameter entsprechend einer vorangehenden Diagnose bestimmt und bei den einzelnen Behandlungssitzungen am Reizstromgerät eingestellt. Daneben sind sogenannte TENS-Geräte bekannt, die mit festen Therapieprogrammen im Rahmen einer Heimtherapie betrieben werden können. Aufgabe der Erdindung ist es, den Indikationsbereich solcher TENS-Geräte zu erweitern. Gemäß der Erfindung ist in einem separaten Therapiefestlegungsgerät (I) eine erste Betriebsschaltung (2) vorgesehen, an welchem bei gleichzeitiger Diagnose die Therapieparameter sowie auch zeitlich festgelegte Therapieprogramme vorwählbar sind und sind die vorgewählten Therapieparameter bzw. die Therapieprogramme speicherbar und vom Therapiefestlegungsgerät (I) auf einen separaten Speicher (49) im eigentlichen Therapiegerät (II) mit zweiter Betriebsschaltung (6) und zugeordneter Sicherheitsschaltung (57) übertragbar. Ein Therapieprogramm läßt sich somit patentenindividuell vorprogrammieren.

FIG 1

EP 0 087 617 A1

Croydon Printing Company Ltd.

0087617

SIEMENS AKTIENGESELLSCHAFT
Berlin und München

Unser Zeichen
VPA 82 P 3707 E

Medizinisches Gerät für die physikalische Therapie, insbesondere elektromedizinisches Reizstromgerät

Die Erfindung bezieht sich auf ein medizinisches Gerät für die physikalische Therapie, insbesondere elektromedizinisches Reizstromgerät zur Behandlung mit elektrischen Impulsen vorgebbarer Parameter, beispielsweise für Folgefrequenz, Impulsbreite und Intensität und/oder Impulsform, bestehend aus einer Betriebsschaltung zur Überwachung der einzelnen Impulse auf Amplitude und/oder Strommenge und wenigstens einer Endstufe.

Speziell Reizstrombehandlungen im Rahmen einer physikalischen Therapie erfolgen bisher zumeist derart, daß vom Therapeuten entsprechend der gestellten Diagnose und seiner Erfahrung Reizströme bestimmter Qualität verordnet wurden, welche dann in Einzelbehandlungssitzungen dem Patienten verabreicht werden. Dazu muß der Patient zu jeder Behandlung in die Praxis des Therapeuten kommen. Aufgrund der Vielzahl der vorgebbaren Reizstromparameter blieb es weitgehend der Erfahrung des Therapeuten überlassen, ob bzw. wie eine optimal wirksame Therapie gefunden wurde.

Neuerdings sind auch Reizstromgeräte auf dem Markt, die als Heimgeräte dem Patienten die Möglichkeit geben sollen, spezifische Therapieformen zu Hause durchführen zu können. Aus Preisgründen einerseits, aber auch aus Sicherheitsgründen andererseits müssen

Wht 5 Rl / 16.02.1982

derartige Geräte vergleichsweise einfach aufgebaut sein und dürfen insbesondere keine hohen Spannungen führen.

Letztere Geräte werden in der Fachwelt    als sogenannte TENS-Geräte (Transkutane Elektrische Nerven- Stimulation) bezeichnet. Derzeit sind zwei Gruppen von TENS-Geräten bekannt: Eine erste Gruppe ist lediglich für einen vorgegebenen Behandlungsstrom ausgebildet; eine zweite Gruppe hat dagegen einstellbare Parameter für Folgefrequenz sowie Pulsbreite und im beschränkten Maße auch für die Reizintensität. Die Kurvenform ist aber im allgemeinen nicht veränderbar.

Aufgrund des vergleichsweise einfachen Aufbaus der TENS-Geräte ist deren Indikationsbereich naturgemäß eng begrenzt. Bringt die Therapie mit einem vorhandenen Gerät nicht den erwarteten Erfolg, so muß jeweils der Gerätetyp  bzw. das Fabrikat gewechselt werden.

Aufgabe der Erfindung ist es daher, ein Gerät für die physikalische Therapie zu schaffen, das nicht die Einschränkungen der obenbeschriebenen TENS- Geräte aufweist, sondern einen möglichst vielseitigen Indikationsbereich hat.

Die Aufgabe wird erfindungsgemäß dadurch gelöst, daß eine erste Betriebsschaltung in einen separaten Therapiefestlegungsgerät vorgesehen ist, an welchem bei gleichzeitiger Diagnose die Therapieparameter sowie auch zeitlich festgelegte Therapieprogramme vorwählbar sind, und daß die vorgewählten Therapieparameter bzw. die Therapieprogramme speicherbar und vom Therapiefestlegungsgerät auf einen separaten Speicher im eigentlichen Therapiegerät mit zweiter Betriebsschaltung und zugeordneter Sicherheitsschaltung übertragbar sind.

Beim Gerät nach der Erfindung sind also ein separates Therapiefestlegungsgerät und das eigentliche Therapiegerät vorhanden. Mit dem universell ausgelegten Therapiefestlegungsgerät wird zunächst vom Therapeuten im Rahmen einer Diagnose untersucht und festgelegt, welche Kurvenform, Pulsfolge bzw. -breite, Intensität und Therapiezeit bei dem zu behandelnden Patienten die optimalen Ergebnisse bringt. Diese individuell für die Behandlung des Patienten festgelegten Parameter werden zwischengespeichert und sind auf einen Speicher des eigentlichen Therapiegerätes übertragbar, von wo die festgelegten Behandlungen jederzeit abrufbar sind.

Die Speicher sind vorzugsweise als digitale Halbleiterspeicher ausgelegt und Teil eines Mikroprozessorsystems zum Einlesen bzw. Auslesen der Behandlungsparameter. Das Therapiegerät hat eine derart ausgelegte Sicherheitsschaltung, daß sie insbesondere auch beim ersten Fehlerfall voll wirksam ist. Zur Bedienung durch den Patienten braucht dieses Therapiegerät lediglich einen Einschalter und gegebenenfalls einen Intensitätsregler für einen eingeengten Einstellbereich von beispielsweise ± 20 % aufzuweisen. Sonstige Bedienelemente sind nicht vorgesehen.

Das erfindungsgemäß ausgebildete Gerät hat gegenüber den bisher verwendeten Geräten wesentliche Vorteile: Beispielsweise können beliebige Therapieprogramme realisiert werden. Dadurch kann das Gerät hinsichtlich der Therapieformen leicht neuen Erkenntnissen angepaßt werden.

Wesentlicher Grundgedanke der Erfindung ist, dem Patienten zwar ein Gerät zur Heimtherapie zur Verfügung zu stellen, allerdings einen Eingriff in die Geräteparameter nur vom Therapeuten vornehmen zu lassen, so daß eine Therapie nach eigenen Vorstellungen des Patienten unmöglich ist. Mit einem solchen Gerät ist also eine mißbräuchliche Verwendung außerhalb ärztlicher Kontrolle ausgeschlossen. Es ist möglich, daß nach vorgegebenen Zeitprogrammen durch Synchronisation mit einer eingebauten Uhr das Gerät lediglich zu vorgegebenen Zeiten und/oder über genau festgelegte Zeitspannen betrieben werden kann.

Daneben kann die erfindungsgemäße Geräteanordnung auch vorteilhaft im Krankenhaus eingesetzt werden. Es wird dabei lediglich ein Therapiefestlegungsgerät benötigt, auf dem vom Therapeuten für unterschiedliche Patienten die erforderlichen Parameter individuell festgelegt werden; eine größere Anzahl von einfachen Therapiegeräten kann dagegen dezentral am Patientenbett verbleiben und von der Krankenschwester oder auch vom Patienten selbst aktiviert werden.

Bei der Erfindung wird also ein vergleichsweise aufwendiges Therapiefestlegungsgerät mit einer Vielzahl jederzeit umprogrammierbarer Geräte kombiniert. Es ergibt sich so ein erheblich verbessertes Kosten/Nutzen-Verhältnis. Durch Programmspeicherung auf geeigneten Datenträgern lassen sich auch in bekannter Weise Programmbibliotheken aufbauen, wodurch sich Therapieerfolge dokumentieren und damit objektivierbar sowie auch gegebenenfalls besser reproduzierbar machen lassen.

Insgesamt ergibt sich also durch die Erfindung eine entscheidende Erweiterung der Indikation insbesondere gegenüber den herkömmlichen TENS-Geräten. Außer der speziellen Anwendung für die Reizstromtherapie kann die Erfindung aber im Rahmen einer allgemeinen physikalischen Therapie für weitere Teilgebiete, beispeilsweise die Magnetfeldtherapie, angewendet werden.

Weitere Einzelheiten und Vorteile der Erfindung ergeben sich aus der nachfolgenden Figurenbeschreibung eines Ausführungsbeipiels anhand der Zeichnung. Es zeigen:

Fig. 1 grob schematisch die erfindungsgemäße Anordnung bestehend aus einem Therapiefestlegungsgerät einerseits und einem Therapiegerät andererseits,

Fig. 2 blockschaltbildmäßig das Therpiefestlegungsgerät und

Fig. 3 blockschaltmäßig das eigentliche Therapiegerät. ·

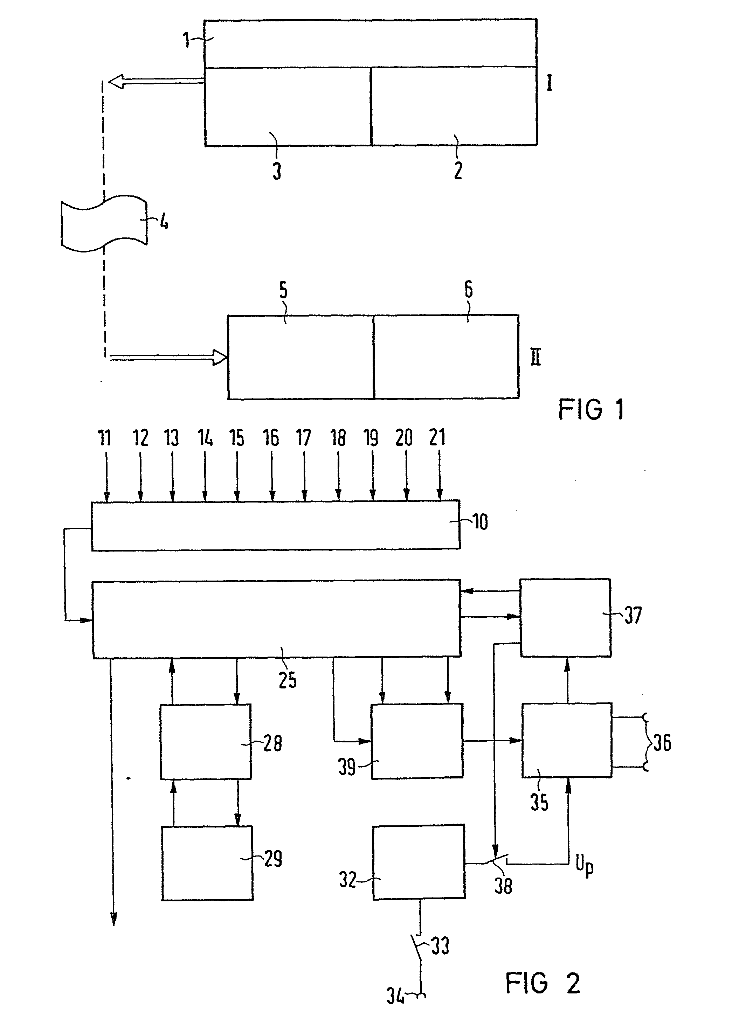

In der Figur 1 bedeutet I ein Therapiefestlegungsgerät, das gleichermaßen zur Diagnose und/oder Therapie verwendbar ist, und II ein eigentliches Therapiegerät.

Das Therapiefestlegungsgerät I beinhaltet ein Eingabefeld 1 zur Festlegung der Therapieparameter, eine Betriebsschaltung 2 mit Patientenstromausgängen sowie eine Mikroprozessoreinheit 3. Diese drei Einheiten sind im Block I jeweils nur angedeutet. Ganz entsprechend besteht das eigentliche Therapiegerät II aus einer zugehörigen Mikroprozessoreinheit 5 und einer Betriebsschaltung 6.

Über einen Datenübertragungsweg sind die Prozessorsysteme der Einheiten I und II miteinander verbindbar. Diese Verbindung kann über eine Leitung, drahtlos oder auch mittels spezieller Datenträger, wie beispielsweise Magnetkarten, erfolgen. Ein derartiger Datenträger ist mit 4 angedeutet.

In der Figur 2 bedeutet 10 eine Eingabetastatur, die beispielsweise als Folientastatur ausgebildet sein kann. Die Eingabetastatur 10 ist von Einstellgliedern 11 bis 21 zur Vorwahl beispielsweise folgender Reizstromparameter ansteuerbar.

|  |  |  |
|---|---|---|
| a) | = | Impulsbreite |
| b) | = | Impulspause |
| c) | = | TENS-Programme |
| d) | = | Ultrareizstrom |
| e) | = | Bernard'sche Ströme |
| f) | = | Rechteck/Dreiecksimpulse |
| g) | = | Faradisation |
| h) | = | Polarität |
| i) | = | Schwellenanstieg |
| k) | = | Intensität |
| l) | = | Therapiezeit |

Mit den beispielhaft angeführten Bezeichnungen für die Therapieparameter kann der Fachmann bestimmte Stromformen definieren; dabei wird insbesondere durch a) - g) die Impulsform charakterisiert. Weitere Parameter sind möglich.

Das Therapiefestlegungsgerät I weist folgende weitere Einheiten auf, die eine Betriebsschaltung einerseits und eine Prozessoreinheit andererseits bilden und über eine Ein/Ausgabeeinheit .25 als Schnittstelle verbunden sind:

Ein Netzteil 32 ist über einen handbetätigbaren
Schalter 33 an das Netz 34 anschließbar. Vom Netzteil 32 wird eine Endstufe 35 angesteuert, welche Ausgänge 36 für den Anschluß von Patientenelektroden
aufweist. Der Endstufe 35 ist eine Sicherheitsschaltung 37 zugeordnet, welche gleichermaßen in Wechselwirkung mit der Ein-/Ausgabeeinheit 25 steht. Abweichungen von eingestellten Sollwerten werden in
der Sicherheitsschaltung 37 erfaßt, wobei gegebenenfalls ein Schalter 38 in der spannungsführenden
Leitung unterbrochen wird. Zwischen Ein-/Ausgabeeinheit 25 und Endstufe 35 ist weiterhin eine Einheit 39 mit der Funktion "An- und Abschwellen" eingeschaltet, welche bewirkt, daß bei vorgegebenen
Therapieparametern die Veränderung der Reizintensität kontinuierlich erfolgt und bei Ein- oder Ausschalten des Gerätes insbesondere keine abrupten
Änderungen des Patientenstromes auftreten.


Mit der so beschriebenen Betriebsschaltung ist also
außer einer Diagnose auch bereits eine Behandlung
eines Patienten möglich, wobei speziell im Rahmen
der Diganose alle relevanten Therapieparameter prüfbar sind. Über einen Mikroprozessor 28 können die
an der Eingabetastatur 10 angewählten Parameter aus
dem Arbeitsspeicher des Prozessorsystems abgerufen
und in entsprechende Stromformen umgesetzt werden.
Die im Rahmen der Diagnose ausgewählten Parameter
werden in einem separaten Programmspeicher 29 eingelesen. Als Speicher werden übliche im Zusammenhang
mit Prozessoren verwendbare Speicher benutzt, beispielsweise ein sog. RAM oder ein EE-PROM, das elektrisch umprogrammiert werden kann.

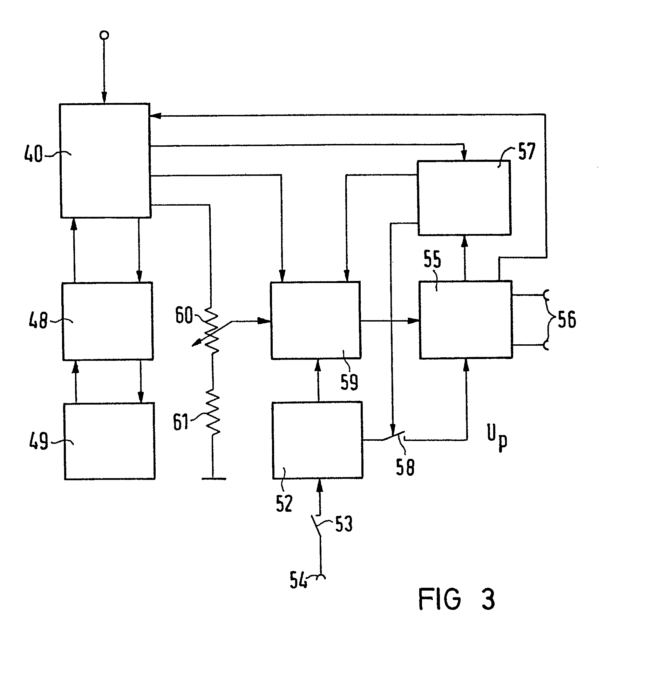

Das Therapiegerät II gemäß Figur 3 weist eine Ein-/ Ausgabeeinheit 40 auf, der ein Mikroprozessor 48 mit Programmspeicher 49 zugeordnet ist, welche letztere den Einheiten 28 und 29 der Figur 2 entsprechen. Das im Therapiefestlegungsgerät I festgelegte und zwischengespeicherte Therapieprogramm wird in den Speicher 49 übertragen und ist daraus über die Prozessoreinheit 48 auslesbar; die Ein-/Ausgabeeinheit 40 wird dabei zur Schnittstelle der Betriebsschaltung im Therapiegerät. Damit ist ein Zugriff zu den Reizstromparametern gegeben, wodurch die Betriebsschaltung entsprechend dem festgelegten Therapieprogramm angesteuert werden kann.

Die Betriebsschaltung der Figur 3 entspricht in den wesentlichen Elementen der Betriebsschaltung nach Figur 2: Ein Netzteil 52 ist über einen handbetätigbaren Netzschalter 53 an das Netz 54 angeschaltet. Vom Netzteil 52 wird mit der Betriebsspannung $U_P$ eine Endstufe 55 angesteuert, welche Ausgänge 56 zum Anschluß der Patientenelektroden aufweist. Der Endstufe 55 ist eine Sicherheitsschaltung 57 zugeordnet, die in Wechselwirkung mit der Ein-/Ausgabeeinheit 40 steht und insbesondere die Patientenspannungen bzw. -ströme mit den vorgegebenen Parametern vergleicht. Bei Abweichungen wird ein Schalter 58 in der Leitung für die Betriebsspannung geöffnet, so daß der Patient sofort von den spannungsführenden Teilen trennbar ist.

Weiterhin ist wiederum eine Einheit 59 für die Funktion "An- und Abschwellen" vorhanden, welche mit der Ein- und Ausgabeeinheit 40 und der Sicherheitsschaltung 57 verbunden ist. Mit dieser Einheit wird bei Beginn und Ende der Therapie die Endstufe 55 kontinuierlich herauf- bzw. heruntergeregelt. Zusätzlich ist der Einheit 59 ein Spannungsteiler mit Widerständen 60 und 61 zugeordnet, von denen der Widerstand 60 zwecks Stromregelung als Potentiometer ausgebildet ist.

Am Therapiegerät II kann also bei einem im Speicher 49 eingelesenen Therapieprogramm durch den Patienten mittels des Potentiometers 60 die Intensität des Reizstromes in einem gewissen Größenbereich verändert werden. Ansonsten sind alle Parameter, beispielsweise auch die Behandlungszeit, durch das Programm festgelegt. Es ist daneben günstig, eine Kontrolleinrichtung vorzusehen, die dem Patienten einen Stromfluß aufzeigt. Eine solche Einrichtung kann Teil der Überwachungseinrichtung sein und spricht nur dann an, wenn tatsächlich ein Therapiestrom fließt. Von der Endstufe 55 besteht weiterhin eine Rückkopplung zum Speicher. Es können damit die tatsächlich durchgeführten Behandlungen mit beispielsweise Therapiezeit und -strom zurückgelesen werden, wodurch dem Therapeuten über die Strommenge als relevanter Größe die Möglichkeit einer direkten Therapiekontrolle gegeben ist.

Die Endstufen 35 bzw. 55 der Reizstromgeräte I und II können alternativ für Konstantstrom bzw. Konstantspannung ausgelegt sein. Mit der Sicherheitsschaltung 37 bzw. 57 ist gewährleistet, daß keine unerwünscht hohen Ströme bzw. Spannungen zum Patientenkörper gelangen. Dabei ist die Sicherheitsschaltung in bekannter Weise derart aufgebaut, daß sie im sog. "ersten Fehlerfall", d.h. bei Ausfall von elektronischen Bauelementen voll wirksam bleibt. Sie kann dafür auch zweifach ausgelegt sein.

Das beschriebene Reizstromgerät hat jeweils eine Ausgangseinheit für einen Ausgangsstromkreis als Teil der Betriebsschaltung. Für eine Interferenzstromtherapie oder auch eine andere Kombinationstherapie kann das gleiche Gerät auch zwei und mehr Ausgangseinheiten für eine entsprechende Zahl von Strömen aufweisen.

Beim Ausführungsbeispiel sind Therapiefestlegungsgerät und Therapiegerät als netzbetriebene Geräteeinheiten ausgelegt. Insbesondere als Therapiegerät kann aber bei Bedarf auch batteriebetrieben sein.

Die Erfindung wurde speziell für die Reizstromtherapie beschrieben. Daneben ist das gleiche Prinzip aber auch im Rahmen anderer Methoden der physikalischen Therapie, bei der eine Vielzahl von Parametern patientenindividuell zu bestimmen und festzulegen sind, verwendbar. Dies gilt beispielsweise auch für die Magnetfeldtherapie.

3 Figuren
13 Patentansprüche

Patentansprüche

1. Medizinisches Gerät für die physikalische Therapie, insbesondere elektromedizinisches Reizstromgerät zur Behandlung mit elektrischen Impulsen vorgebbarer Parameter, beispielsweise für Folgefrequenz, Impulabreite und Intensität und/oder Impulsform, bestehend aus einer Betriebsschaltung zur Überwachung der einzelnen Impulse auf Amplitude und/oder Strommenge und wenigstens einer Endstufe, d a d u r c h  g e k e n n z e i c h n e t , daß eine erste Betriebsschaltung (2) in einen separaten Therapiefestlegungsgerät (I) vorgesehen ist, an welchem bei gleichzeitiger Diagnose die Therapieparameter sowie auch zeitlich festgelegte Therapieprogramme vorwählbar sind, und daß die vorgewählten Therapieparameter bzw. die Therapieprogramme speicherbar und vom Therapiefestlegungsgerät (I) auf einen separaten Speicher (49) im eigentlichen Therapiegerät (II) mit zweiter Betriebsschaltung (6) und zugeordneter Sicherheitsschaltung (57) übertragbar sind.

2. Medizinisches Gerät nach Anspruch 1, d a d u r c h  g e k e n n z e i c h n e t ,  daß das Therapiegerät (II) von einer Prozessoreinheit zum Einlesen der Programme in den Speicher (49) bzw. Auslesen aus dem Speicher (49) gesteuert ist.

3. Medizinisches Gerät nach Anspruch 1, d a d u r c h  g e k e n n z e i c h n e t ,  daß das Therapiefestlegungsgerät (I) von einer Prozessoreinheit zum Abrufen von Therapieparametern aus einem Speicher (29) und Einlesen von Therapieprogrammen in den Speicher (29) gesteuert ist.

4. Medizinisches Gerät nach einem der vorliegenden Ansprüche, dadurch gekennzeichnet, daß die Speicher (29, 49) digitale Halbleiterspeicher sind.

5. Medizinisches Gerät nach Anspruch 4, dadurch gekennzeichnet, daß die digitalen Halbleiterspeicher (29, 49) ein RAM oder ein EE-PROM sind.

6. Medizinisches Gerät nach einem der vorhergehenden Ansprüche, dadurch gekennzeichnet, daß die Überwachungsschaltung (37, 57) als Sicherheitseinrichtung zum kontinuierlichen Istwert-/Sollwert-Vergleich des Patientenstroms ausgelegt ist.

7. Medizinisches Gerät nach Anspruch 6, dadurch gekennzeichnet, daß die Überwachungsschaltung (37, 57) als Sicherheitseinrichtung zweifach ausgelegt ist.

8. Medizinisches Gerät nach Anspruch 1, dadurch gekennzeichnet, daß das Therapiegerät (II) ein Potentiometer (60) zur Veränderung der Reizstromintensität in vorgebebenem Größenbereich aufweist.

9. Medizinisches Gerät nach Anspruch 1, dadurch gekennzeichnet, daß die von der Ausgangsstufe (55) im Therapiegerät (II) an den Patienten abgegebene Strommenge in den Speicher (49) zurücklesbar ist.

10. Medizinisches Gerät nach einem der vorhergehenden Ansprüche, dadurch gekennzeichnet, daß die Endstufe (35, 55) eine Konstant-Stromstufe ist.

11. Medizinisches Gerät nach einem der vorhergehenden Ansprüche, dadurch gekennzeichnet,

daß die Endstufe (35, 55) eine Konstant-Spannungsstufe ist.

12. Medizinisches Gerät nach einem der vorhergehenden Ansprüche, d a d u r c h   g e k e n n z e i c h ‑ n e t ,   daß der Endstufe (55) im Therapiegerät (I) ein Netzteil (53) zum Netzanschluß zugeordnet ist.

13. Medizinisches Gerät nach einem der vorhergehenden Ansprüche, d a d u r c h   g e k e n n z e i c h ‑ n e t ,   daß der Endstufe (55) im Therapiegerät (I) eine Batterie zur Spannungsversorgung zugeordnet ist.

FIG 1

FIG 2

FIG 3

Europäisches
Patentamt

EUROPÄISCHER RECHERCHENBERICHT

**0087617**
Nummer der Anmeldung

EP 83 10 1151

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | FR-A-1 213 080 (GUERINEAU) <br> * Seite 2, linke Spalte, Zeilen 17-39; rechte Spalte, Abschnitte 3-6; Seite 3, Abschnitt 6 * | 1 | A 61 N 1/08 |
| A | FR-A-2 137 352 (RETTEL) <br><br> * Seite 1, Zeilen 4-28, Zeilen 33,34 * | 1,13, 14 | |
| A | DE-A-2 903 392 (REINHOLD) <br><br> * Seite 5 * | 1,2,4, 5 | |
| A | US-A-4 195 626 (SCHWEIZER) <br> * Spalte 1, Zeile 66 - Spalte 2, Zeile 20; Spalte 5, Zeilen 30-39; Spalte 6, Zeilen 22-26, Zeilen 39-41; Spalte 10, Zeilen 22-28, Zeilen 50-55 * | 2-6,9 | RECHERCHIERTE SACHGEBIETE (Int. Cl. ³) <br><br> A 61 N |
| A | EP-A-0 026 324 (SIEMENS) <br> * Seite 3, Zeilen 13-22 * | 6,8,11 | |
| A | DE-A-2 133 999 (HARTWICH) <br><br> ----- | | |

Der vorliegende Recherchenbericht wurde für alle Patentansprüche erstellt.

| Recherchenort <br> DEN HAAG | Abschlußdatum der Recherche <br> 19-05-1983 | Prüfer <br> SIMON J.J.E. |
|---|---|---|